# EUROPEAN PATENT APPLICATION

(11) **EP 4 431 031 A1**
(43) Date of publication of application: **18.09.2024**
(21) Application number: 24163713.1
(22) Date of filing: 15.03.2024
(51) Int. Cl.: A61B 17/122

(54) **SURGICAL CLAMP INSERT**

(30) Priority: 15.03.2023 US 202318121863
(71) Applicant: Vitalitec International, Inc., Plymouth, MA 02360 (US)
(72) Inventor: BERTSCH, Karrie, Los Gatos, CA 95032 (US)
(74) Representative: Patentanwälte und Rechtsanwalt Weiß, Arat & Partner mbB

(57) **Abstract**

An insert for a surgical clamp includes an integral structure having a spine, a connecting web, and a contact portion, wherein the spine is an elongate structure having a lower surface defining structure to engage a jaw of the clamp; the contact portion has a textured surface configured to contact tissue; and the web extends between an upper surface of the spine and the contact portion and flexibly supports the contact portion above the upper surface of the spine. An overmold can be applied to the integral structure to surround the web and parts of the contact portion with the textured surface exposed through the overmold.

## Description

### BACKGROUND OF THE INVENTION

The invention relates to surgical clamps and, more particularly, to an insert for a surgical clamp and method for making same.

Surgical clamps exist in many sizes with many different types of clamp shapes (e.g., curved jaws, straight jaws, etc.). In addition, many different types of jaw surfaces exist, as adapted to the specific function performed by the clamp. When a different function is to be performed, one must either use a different clamp, or in some circumstances replaceable pads may be added to the jaws.

Many existing surgical clamps have jaws with hard clamping surfaces. Some replaceable pads for these clamps are designed to fit over the jaws to provide a softer or otherwise different clamping surface. However, these pads are often bulky, reducing the sleekness of the clamp and jaws. In addition, these pads are typically designed to fit over only straight jaws and are generally straight themselves. There is a need for other shapes such as curved or S-shaped.

Other existing surgical clamps have curved, replaceable pads that are sleek, but these sleek pads are not soft and may be inappropriate for many applications.

Still other existing surgical clamps have soft pads but these pads are not replaceable. This makes the pads harder to clean. Autoclaving may cause soft or delicate pads to deteriorate or wear out more quickly, with the result that the pad surfaces may become less soft or less delicate. As an alternative, the pad surfaces may be constructed to be less soft or less delicate in order to have a longer lifetime.

Some surgical clamps (e.g., U.S. Pat. No. 3,503,398) have replaceable pads that are soft, but have other concerns. For example, some pads can slip once installed. Decreasing the possibility of the pads slipping off may increase the effort necessary to install the pads. In addition, the portion of the pad that attaches to the clamp may not be flexible.

Finally, some existing surgical clamps have replaceable pads that are not tightly secured to the jaws. With such clamps, the pads may move laterally after the vessel or tissue has been clamped. This lateral movement makes for an insecure clamp subject to wobbling, that may shear or tear the vessel or tissue being clamped. A solution to many of these issues is addressed in US Patent 6,228,104 to Fogarty et al. and related patents, all assigned to the assignee of the present application.

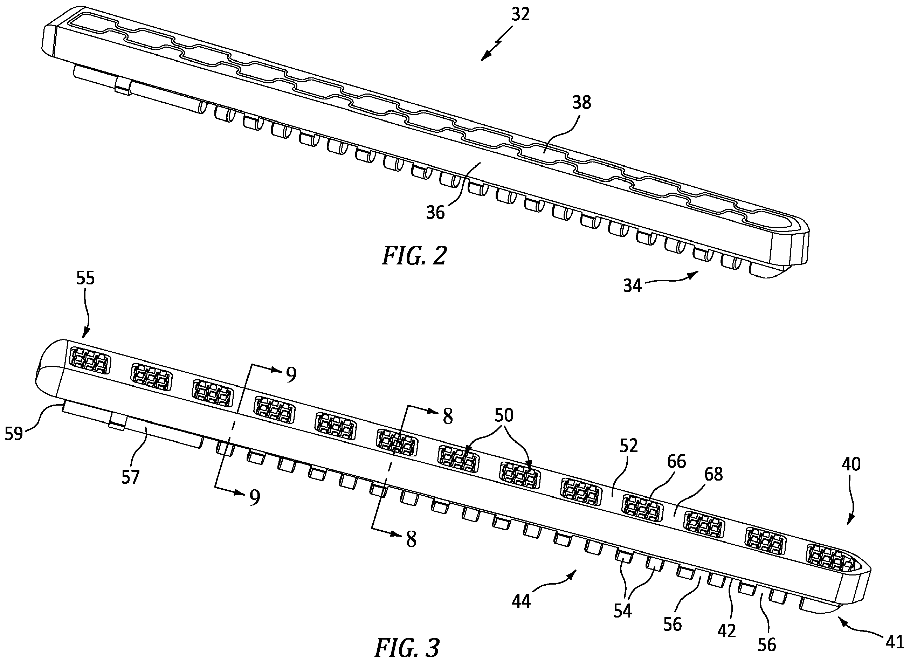

Further, US Patent 10,368,887, also granted to the assignee of the present application, discloses a particularly effective replaceable pad which addresses the above issues. This patent discloses a device that works very well. It securely grips vessels using micro-hook material such that excessive clamping force is not needed for the vessel to remain occluded and the clamp to stay in place. This existing device is molded three times, an original mold with two overmolds, and is then assembled. In this process, first, the spine of the insert is molded. Next, a slightly harder durometer elastomer is overmolded onto the spine. The elastomer needs to be hard so that it does not tear off the spine easily. Another overmold step then takes place to place a soft elastomer cover over the first overmold so that it conforms to the vessel. Last, a die-cut piece of purchased micro-hook material is bonded to the insert. This requires cutting down the micro-hook material to the proper size and shape, priming, gluing and precise placement within the recess in the overmold structure. The final insert is shown in FIG. 2 which will be further discussed below.

Unfortunately, the process for making this insert is very expensive and time consuming. Thus, an area where further improvement can be made is with respect to the structure and manufacture of the pad.

### SUMMARY OF THE INVENTION

The present disclosure relates to a clamp insert having a simplified structure that nevertheless functions as desired such that an insert can be produced that performs as well or better than the known insert, while the method for making the insert requires fewer steps and parts and much less time consuming labor.

In one non-limiting embodiment, an insert for a surgical clamp, comprises an integral structure having a spine, a connecting web, and a contact portion, wherein the spine is an elongate structure having a lower surface defining structure to engage a jaw of the clamp; the contact portion has a textured surface configured to contact tissue; and the web extends between an upper surface of the spine and the contact portion and flexibly supports the contact portion above the upper surface of the spine.

In a non-limiting configuration, the web comprises a series of columns connected between the upper surface of the spine and a lower surface of the contact portion.

In a further non-limiting configuration, the contact portion comprises an elongate structure comprising a series of laterally extending sections defining contact surfaces having gripping contours, and intervening laterally narrower connecting sections between the laterally extending sections.

In a still further non-limiting configuration, the columns connect between the upper surface of the spine and a lower surface of the laterally narrower connecting sections, whereby each laterally extending section extends between adjacent columns whereby the laterally extending section has vertical flexibility relative to the spine. Further, the insert can comprise an overmold surrounding at least the web and portions of the contact portion, the overmold having a series of windows aligned with the laterally extending sections of the contact portion.

In another non-limiting configuration, the insert further comprises an overmold surrounding at least the web and portions of the contact portion, the overmold having a series of windows, and the contact portion having a series of textured surface sections exposed through the series of windows.

In still another non-limiting configuration, the web defines lateral gaps, and the overmold extends through the lateral gaps to engage the spine.

In a further non-limiting configuration, the overmold comprises an elastomeric material having a durometer of between 20 and 65 Shore A.

In a still further non-limiting configuration, the overmold comprises a silicone material.

In another non-limiting configuration, the structure for engaging the jaw of the clamp comprises a series of engagement segments separated by gaps.

In still another non-limiting configuration, the structure for engaging the jaw of the clamp further comprises a distal engagement segment positioned at a distal end of the lower portion of the spine.

In a further non-limiting configuration, the distal engagement segment is longer than the engagement segments, and has a flat distal end, whereby the flat distal end snaps into place when inserted into a jaw channel.

In a still further non-limiting configuration, the spine, the web and the contact portion are a single unitary component.

In another non-limiting configuration, the spine, the web and the contact portion comprise the same material.

In another non-limiting embodiment, a clamp surface for an insert of a surgical clamp, comprises an elongate spine; a plurality of contact sections positioned along the elongate spine; and a web portion extending between the elongate spine and spaced edges of the contact sections to flexibly support the contact sections above the elongate spine.

In one non-limiting configuration, the elongate spine defines a longitudinal axis, the contact sections are arranged along the longitudinal axis with connection portions extending therebetween, and the web portion comprises a series of columns extending between the upper surface of the spine and the narrower connection portions.

In another non-limiting configuration, the connection portions are laterally narrower than the contact sections.

In a further non-limiting embodiment, a method for making an insert for a surgical clamp, comprises forming an integral structure having a spine, a connecting web, and a contact portion, wherein the spine is an elongate structure having a lower surface defining structure to engage a jaw of the clamp; the contact portion has a textured surface configured to contact tissue; and the web extends between an upper surface of the spine and the contact portion and flexibly supports the contact portion above the upper surface of the spine; and forming an overmold around at least the connecting web and a portions of the contact portion while leaving the textured surface exposed.

In a non-limiting configuration, the step of forming the overmold comprises forming the overmold having a series of windows through which the textured surface is exposed.

In a further non-limiting configuration, the textured surface comprises a series of textured surfaces defined along a length of the contact portion, and the series of windows are arranged along a length of the overmold to coincide with the series of textured surfaces.

In a still further non-limiting configuration, the integral structure has openings between the contact portion and the spine, and the step of forming the overmold comprises forming the overmold through the openings.

In another non-limiting configuration, the steps of forming the integral structure and forming the overmold comprise injection molding the integral structure, and then injection molding the overmold.

The foregoing features and elements may be combined in various combinations without exclusivity, unless expressly indicated otherwise. These features and elements as well as the operation thereof will become more apparent in light of the following description and the accompanying drawings. It should be appreciated that the following description and drawings are intended to be exemplary in nature and non-limiting.

### BRIEF DESCRIPTION OF THE DRAWINGS

A detailed description of non-limiting embodiments of the present disclosure follows, with reference to the attached drawings, wherein:
FIG. 1 shows a surgical clamp with pads or inserts;
FIG. 2 is a perspective view of a known insert for a surgical clamp;
FIG. 3 is a perspective view of an insert according to the present invention;
FIG. 4 is a perspective view of a spine component of the insert of FIG. 3;
FIG. 4A is a cross section taken through a typical jaw structure of a clamp and shows a channel that runs longitudinally through at least a portion of the jaw;
FIG. 5 is an enlarged view of one end of the spine component of FIG. 4;
FIG. 6 is an enlarged view of the other end of spine component of FIG. 4;
FIG. 7 is an enlarged side view of a portion of the spine component of FIG. 4;
FIG. 8 is a cross section taken along the lines 8-8 of FIG. 4;
FIG. 9 is a cross section taken along the lines 9-9 of FIG. 4; and
FIG. 10 illustrates a further embodiment with respect the grip structure of the contact surface of the insert.

### DETAILED DESCRIPTION

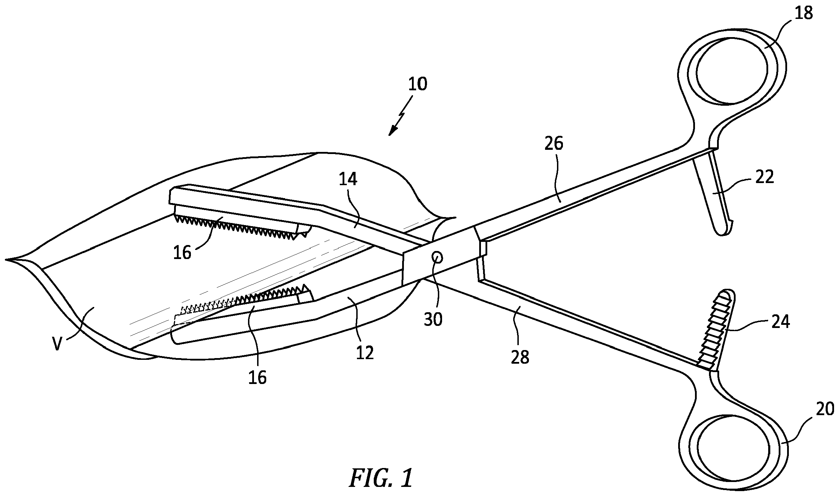

FIG. 1 shows an exemplary surgical clamp 10 having jaws 12, 14 each including a pad 16. The clamp 10 is shown in the process of clamping a vessel V. It may also be used to clamp organs or other bodily tissue.

The clamp 10 can include finger and thumb rings 18, 20 for operating the clamp. A pawl 22 and ratchet teeth 24 can be provided to lock the clamp 10 when it is applied to vessel V. Clamp 10 can be defined by pivotable members 26, 28 which can be pivotally connected by pin 30. Jaws 12, 14 apply a clamping force to vessel V, as cushioned by pads 16.

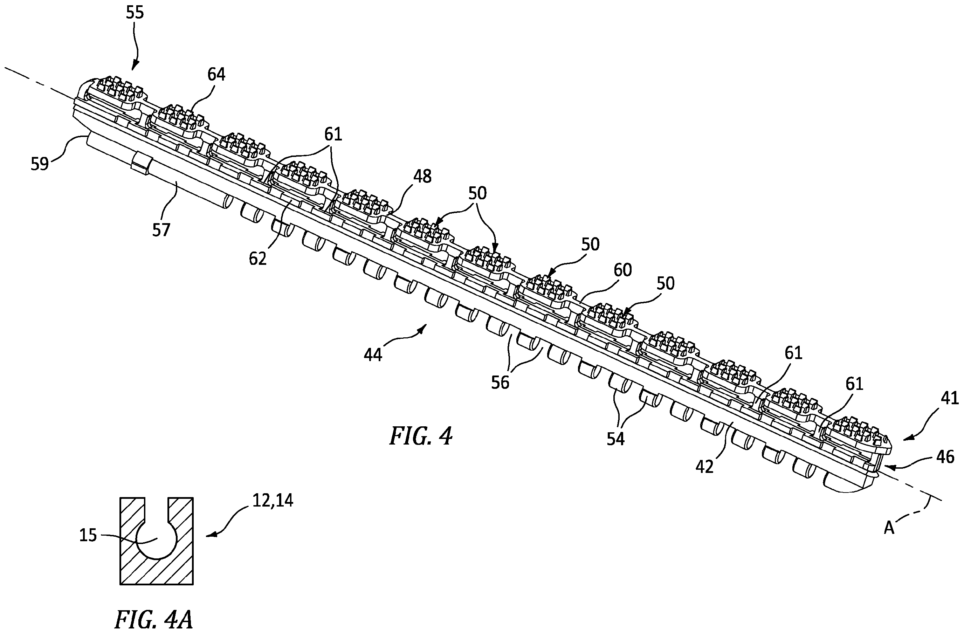

Jaws 12, 14 can have longitudinal channels (15, FIG. 4a, discussed further below) which run at least the portion of the length of the jaws which is to be cushioned by pads 16. This distance may be, for example, approximately 33mm, 66mm or 86mm from the distal end of the jaws. Pad 16 has an engaging structure (further discussed and illustrated below) which can be inserted into the channel of jaws 12, 14 to hold the pads securely in place for use in a surgical procedure while allowing the pad to be removed for changing the function of the clamp and/or sterilizing the clamp while disposing of a used pad.

FIG. 2 illustrates a known insert 32 which has a base or spine 34, a first overmold of a hard material (not visible in FIG. 2) to securely grip the spine 34, a second overmold 36 of a softer material designed to conform to tissue to be clamped with the insert, and a layer or sheet of micro hook material 38 which is secured to the second overmold 36. In addition to the multiple overmolding steps, the micro hook material is typically purchased in sheets and then die cut or even manually cut to the desired shapes, and then glued to the second overmold 36. Thus, the structure of FIG. 2 requires at least 6 steps to manufacture, specifically, (1) mold the spine, (2) add first overmold, (3) add second overmold, (4) cut micro hook material to the proper size, (5) apply primer (6) apply glue and (7) adhere the micro hook material to the second overmold.

In accordance with the present disclosure, an insert 40 (FIG. 3) is fabricated of two parts, in a two-step process, which greatly improves efficiency and cost of manufacture. Further, the integral nature of the components of insert 40 can be equal to or improved in terms of performance and reliability of the insert during use as compared to known inserts. As shown in FIGS. 3 and 4, insert 40 comprises an integral structure 41 (FIG. 4) which forms one of the two parts, and which has an elongate base or spine 42 which defines engagement structures 44 along a lower portion for engaging with the jaws of a clamp device. The integral structure 41 also has a web portion 46 (FIG. 4) and a contact portion 48 defining textured surfaces 50 for contacting tissue. Web portion 46 extends between spine 42 and contact portion 48.

Still referring to FIG. 3, a second component of insert 40 is an overmold 52 which surrounds an upper portion of the integral structure, for example web 46 and part of contact portion 48, and defines a suitable surface around textured surfaces 50 for contacting tissue during use.

Overmold 52 can be formed from any of numerous different materials, for example elastomer materials suitable for contact with tissues to which the clamp will be applied. In one non-limiting configuration, overmold 52 can be made from an elastomeric material having a durometer of between 20 and 65 Shore A. This material could also be silicone material.

Referring to FIGS. 4 and 4A, integral structure 41 is illustrated in FIG. 4 without overmold 52 in order to better illustrate the features of structure 41. FIG. 4A is a cross section taken through a typical jaw structure 12, 14 of a clamp 10 and shows a channel 15 that runs longitudinally through at least a portion of the jaw. Inserts have engagement structure that can be engaged into channel 15 to hold the insert in place. FIG. 4A is a simplified cross section view of this structure, and the actual shape and structure of the jaw may be different and/or have additional features.

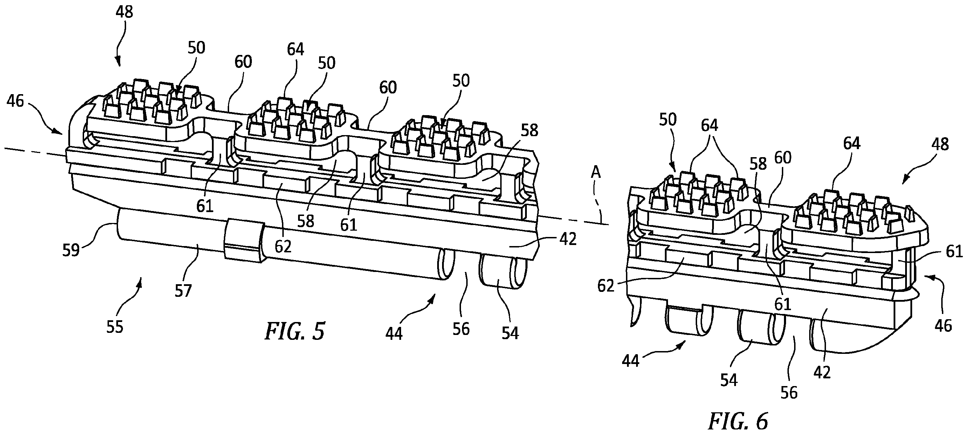

In FIG. 4, the engagement structure 44 as shown in this embodiment is a series of engagement segments 54 that can, for example, be substantially cylindrical in shape (see also FIGS. 5 and 6), and defined along an axis A of spine 42, and extending downwardly from spine 42. In the embodiment illustrated, there are a plurality or series of cylindrical members as engagement segments 54 arranged along axis A and separated by gaps 56. Gaps 56 help to give insert 40 flexibility which can help during installation of an insert by sliding cylindrical members along channel 15. Further, these gaps 56 give the insert flexibility in the event insert 40 is to be installed into a jaw that is not straight, as some clamps have jaws that are laterally and/or vertically curved to define specific clamping surfaces for different applications.

The shapes of engagement segments 54 and channel 15 are selected so that the engagement segments can be entered into the channel and moved along the channel to a use position where the engagement segments 54 will be securely held within channel 15. In this regard, one end 55 of integral member 41 has an end engagement segment 57 that is configured to be longer (along axis A) than the other engagement segments 54. Further, end engagement segment 57 can have a flat distal most facing surface 59. End engagement segment 57 can be longer than the other engagement segments 54. This is useful since end engagement segment 57 is the last segment to enter channel 15 during installation, and the extra length can create a notable snap when entering the channel, thus confirming that the insert has been completely installed. Further, flat distal most facing surface 59 can engage a flat end wall (not shown) of channel 15 and hold insert 40 in place against axial slipping relative to channel 15.

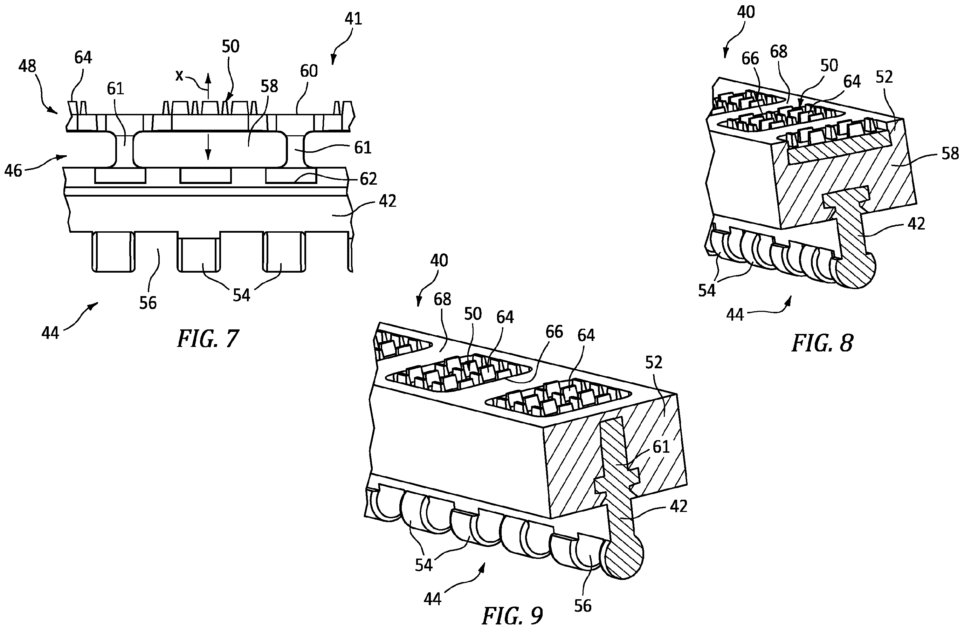

Still referring to FIGS. 4-6, spine 42 of the integrated structure 41 is a generally elongate structure defined along axis A and extending laterally from axis A sufficiently that insert 40 is wide enough to cover the full width of a jaw 12, 14 of clamp 50. Further, web 46 as shown in FIGS. 4-6 can be defined by a series of columns 61 (also FIG. 7) formed along an upper surface of spine 42 and separated by cushion gaps 58 (Fig. 5) which will be further discussed below. Columns 61 extend upwardly from a top surface of spine 42 to support contact portion 48. In the embodiment illustrated, contact portion 48 includes textured surfaces 50 spaced along axis A and connected by narrower connecting portions 60. Columns 61 extend to connecting portions 60 such that each textured surface 50 is vertically supported above spine 42 by a column 61 at each axially arrange edge. This positions each textured surface 50 over a cushion gap 58 which is desirable as this allows textured surfaces to move vertically relative to spine 42 and thereby conform to tissues being clamped. This movement is schematically illustrated at arrows X in FIG. 7. Cushion gaps 58 are also advantageous during manufacture as will be further discussed below.

As best seen in FIGS. 5 and 6, spine 42 has additional crenelated structure 62 extending along lateral edges of spine 42 as shown. These crenelated structures 62 serve to provide additional flexibility to spine 42 such that spine 42 can laterally flex to conform to non-linear jaws. It should be appreciated that although referred to as crenelated, structures 62 do not need to have the specific squared structure as illustrated, and could be rounded or otherwise shaped as well. The main purpose of these structures is to define axially spaced narrower profiles of spine 42 to provide lateral flexibility.

Still referring to FIGS. 4-7 collectively, connecting portions 60 between textured surfaces 50 of contact portion 48 also serve to provide flexibility to this portion of the integrated structure as well, as lateral flexibility is provided by the narrower profile of these portions 60.

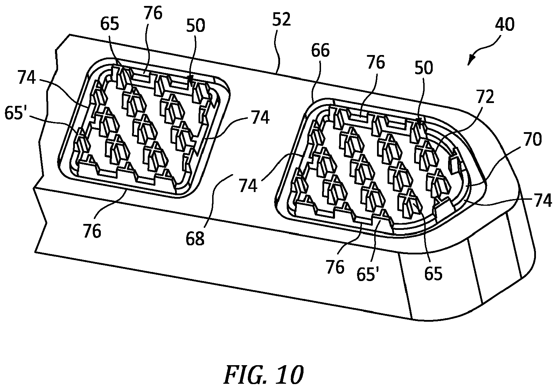

In the drawings, textured surfaces 50 are shown with a plurality of grip structures 64. These grip structures can be provided as tapered protrusions, each having some width, and some arranged at angles relative to others, for example 90-degree angles as shown, in order to provide good gripping of tissues as desired. These structures can have height and density selected according to the use to which the clamp with insert is to be applied, but generally the height of the grip structures 64 will be between 0.005 and 0.040 inches. These gripping structures can also have different shapes, one non-limiting example being a round column and another non-limiting example being a cross or plus(+)- shaped grip which can improve overall strength. Such a cross or plus-shaped grip structure is shown in FIG. 10 and is further described below. Given that integral structure 41 is to be injection molded, preferable in a single injection molding step, the material from which integral structure 41 is manufactured should be selected to be both mechanically stable for the spine and web, and also suitable for contacting tissue at the textured surfaces. In this regard, suitable materials for structure 41 include but are not limited to polypropylene, polyester or other members of the polyolefin family.

FIGS. 5 and 6 illustrate opposite ends of insert 40. In FIG. 5, a rear or last inserted end of insert 40 can be provided having a squared off or rounded squared off structure. In many devices, this will be the end that corresponds to the distal or leading end of the jaws. FIG. 6 shows a front or first-inserted portion of the insert, and can have a structure forming an arrow or other direction indicator to help instruct a technician or surgeon in proper installation of the insert into channels of the jaws.

As set forth above, overmold 52 is positioned over an upper portion of integral structure 41. This is advantageously done in an injection molding procedure such that material of overmold 52 can embrace and fully engage structure 41 to provide insert 40 with mechanical stability to help prevent the insert from coming apart during installation or use. FIGS. 8 and 9 are cross sections taken through insert 40 along the lines 8-8 and 9-9 from FIG. 3. FIG. 8 is taken through a cushion gap 58 to illustrate that material of overmold 52 extends through gap 58 and also surrounds or embraces all of web 46 and columns 61 as well as around side edges of textured surfaces 50. Further, overmold material also engages with crenelated structures 62. This complex contact between overmold 52 and underlying web, spine and contact portions helps to securely hold overmold 52 in place relative to the integral structure 41. As shown in FIG. 8, material of overmold 52 extends laterally through cushion gap 58. Referring to FIG. 9, as shown, column 61 extends upwardly from spine 42, surrounded by overmold 52, to a connecting portion 60 of contact portion 48.

As mentioned above, grip structures 64 can have different shapes. In FIGS. 5 and 6, for example, they are shown as pairs of angled flat upward projections which can be angled at about 90 degrees relative to each other, with a small gap where they would otherwise meet. These pairs can be arranged facing each other, as shown, such that the roughly 90 degree angle defined by the pair of upward projections faces toward another angle defined by another pair of upward projections. FIG. 10 shows another embodiment wherein the grip structures are formed as cross or plus-shaped grip structures 65. As shown, plus-shaped grip structures 65 can have a central structure with 4 laterally extending arms to define the plus-shape. Of course, other numbers of arms could be formed if desired. For example, the grip structure could be formed with 3 arms or 5 or more arms, depending upon the acceptable complexity in the molding process and the desired amount of flexibility and gripping strength. It should also be noted that a perimeter can be formed around the textured surface 50 defined by grip structure 64, 65, and this is also shown in FIG. 10 as a raised wall 70 defining a perimeter around the grip structures of a textured surface 50, and grip structures can be formed along raised wall 70 as well, in this case in the form of partial plus-shaped grip structures 65 having only three arms, in this case two arms extending along raised wall 70 and one extending inwardly into textured surface 50. Further, as shown in FIG. 10, arms 72 extending inwardly from raised wall can have different lateral lengths. In the illustrated embodiment, arms 72 extending from grip structures 65 that are aligned on axial facing walls 74 of raised wall 70 can be shorter than arms extending from lateral facing walls 76 of raised wall 70.

Overmold 52 is generally formed having a width at least matching, preferably exceeding, the width of spine 42 such that all portions of the insert except for textured surfaces 50 which could come in contact with tissue during use are covered by overmold 52. Also, and as discussed above, overmold 52 can be formed with windows or openings 66 which are aligned with textured surfaces 50 as shown in FIG. 3, such that the actual contact surface of insert 40 is defined by textured surfaces 50 and upper surfaces 68 of overmold 52. Windows 66 can be but do not have to be in-line or the same size. Further, structures defining the windows do not need to have the same texture surfaces either. For example, upper surfaces could have texture such as small cross-hatch ribs or the like. Further still, the windows do not need to be complete individual closed-in openings, and instead can be open along the length of the connecting sections as well, similarly to the structure and configuration of the known insert of FIG. 2.

As should be appreciated from the foregoing, insert 40 can be manufactured in 2 steps, namely forming integrated structure 41, and then forming overmold 52 onto structure 41. This avoids the multiple overmolding steps needed to manufacture the insert of FIG. 2, and also completely avoids the separate cutting and gluing of micro hook material.

As indicated above, fabrication of structure 41 can be a single injection molding process, where spine 42, engagement structure 44, web 46 and contact portions 48 with textured surfaces 50 can be fabricated in a single step. Then, overmold 52 can be applied in a second step, also typically an injection molding step, to define insert 40 as disclosed herein.

Although particular steps and sequences are shown, described, and claimed, it should be appreciated that steps may be performed in any order, separated or combined unless otherwise indicated, and will still benefit from the present disclosure.

The foregoing description is exemplary rather than defined by the limitations within. Various non-limiting embodiments are disclosed herein, however, one of ordinary skill in the art would recognize that various modifications and variations in light of the above teachings will fall within the scope of the appended claims. It is therefore to be appreciated that within the scope of the appended claims, the disclosure may be practiced other than as specifically described. Thus, the scope of the present claims is not specifically limited by the details of specific embodiment disclosed herein, but rather the claims define the full and reasonable scope of the invention.

## Claims

1. An insert for a surgical clamp, comprising:
an integral structure having a spine, a connecting web, and a contact portion, wherein
the spine is an elongate structure having a lower surface defining structure to engage a jaw of the clamp;
the contact portion has a textured surface configured to contact tissue; and
the web extends between an upper surface of the spine and the contact portion and flexibly supports the contact portion above the upper surface of the spine.

2. The insert of claim 1, wherein the web comprises a series of columns connected between the upper surface of the spine and a lower surface of the contact portion.

3. The insert of claim 2, wherein the contact portion comprises an elongate structure comprising a series of laterally extending sections defining contact surfaces having gripping contours, and intervening laterally narrower connecting sections between the laterally extending sections.

4. The insert of claim 3, wherein the columns connect between the upper surface of the spine and a lower surface of the laterally narrower connecting sections, whereby each laterally extending section extends between adjacent columns whereby the laterally extending section has vertical flexibility relative to the spine.

5. The insert of claim 1, further comprising an overmold surrounding at least the web and portions of the contact portion, the overmold having a series of windows, and the contact portion having a series of textured surface sections exposed through the series of windows.

6. The insert of claim 5, wherein the web defines lateral gaps, and wherein the overmold extends through the lateral gaps to engage the spine.

7. The insert of claim 1, wherein the structure for engaging the jaw of the clamp comprises a series of engagement segments separated by gaps.

8. The insert of claim 1, wherein the structure for engaging the jaw of the clamp further comprises a distal engagement segment positioned at a distal end of the lower portion of the spine.

9. The insert of claim 8, wherein the distal engagement segment is longer than the engagement segments, and has a flat distal end, whereby the flat distal end snaps into place when inserted into a jaw channel.

10. The insert of claim 1, wherein the spine, the web and the contact portion are a single unitary component.

11. The insert of claim 1, wherein the spine, the web and the contact portion comprise the same material.

12. A clamp surface for an insert of a surgical clamp, comprising:
an elongate spine;
a plurality of contact sections positioned along the elongate spine; and
a web portion extending between the elongate spine and spaced edges of the contact sections to flexibly support the contact sections above the elongate spine.

13. The clamp surface of claim 12, wherein the elongate spine defines a longitudinal axis, and wherein the contact sections are arranged along the longitudinal axis with connection portions extending therebetween, and wherein the web portion comprises a series of columns extending between the upper surface of the spine and the narrower connection portions.

14. A method for making an insert for a surgical clamp, comprising:
forming an integral structure having a spine, a connecting web, and a contact portion, wherein the spine is an elongate structure having a lower surface defining structure to engage a jaw of the clamp; the contact portion has a textured surface configured to contact tissue; and the web extends between an upper surface of the spine and the contact portion and flexibly supports the contact portion above the upper surface of the spine; and
forming an overmold around at least the connecting web and a portions of the contact portion while leaving the textured surface exposed.

15. The method of claim 14, wherein the step of forming the overmold comprises forming the overmold having a series of windows through which the textured surface is exposed, wherein the textured surface comprises a series of textured surfaces defined along a length of the contact portion, and wherein the series of windows are arranged along a length of the overmold to coincide with the series of textured surfaces, wherein the integral structure has openings between the contact portion and the spine, and wherein the step of forming the overmold comprises forming the overmold through the openings.
